Europäisches Patentamt

European Patent Office

Office européen des brevets

(19)

(11) Publication number: **0 351 892**
**A2**

(12) # EUROPEAN PATENT APPLICATION

(21) Application number: 89116798.3

(51) Int. Cl.⁴: **G01N 27/28 , C12M 1/40**

(22) Date of filing: 08.05.84

(30) Priority: 05.05.83 GB 8312262
05.05.83 GB 8312261
06.09.83 GB 8323799
16.12.83 GB 8333644
11.01.84 GB 8400650
29.02.84 GB 8405262
29.02.84 GB 8405263

(43) Date of publication of application:
**24.01.90 Bulletin 90/04**

(60) Publication number of the earlier application in accordance with Art.76 EPC: **0 127 958**

(84) Designated Contracting States:
**BE CH DE FR GB IT LI NL SE**

(71) Applicant: **MediSense, Inc.**
**128 Sidney Street**
**Cambridge Massachusetts 02139(US)**

(72) Inventor: **Hill, Hugh Allen Oliver**
**Nine Clover Close**
**Oxford OX2 9JH(GB)**
Inventor: **Higgins, Irving John**
**Lion Fields Barford Road**
**Wilden Bedford MK43 2QC(GB)**
Inventor: **McCann, James Michael**
**33 Dewhurst Road**
**West Kensington London W14 0ES(GB)**
Inventor: **Davis, Graham**
**Six Heron Heights**
**Goldington Green Bedfordshire(GB)**
Inventor: **Treidl, Bernhard Ludwig**
**18 Medfield Street**
**Boston Mass.(US)**
Inventor: **Birket, Nigel Norman**
**2 Astley Close Sutton**
**Ely Cambridgeshire CB6 2PG(GB)**
Inventor: **Plotkin, Elliot Verne**
**14 George Street**
**Bedford, MK4 3SG(GB)**
Inventor: **Zwanziger, Ron**
**45 Longwood Avenue No.608**
**Brookline, MA 02146(US)**

(74) Representative: **Ruffles, Graham Keith et al**
**MARKS & CLERK 57-60 Lincoln's Inn Fields**
**London WC2A 3LS(GB)**

(54) **Diagnostic aid and assemblies therefor.**

(57) An assembly of circuitry and display means for use in producing a readout value as a diagnostic aid in medicine comprises:

(a) a pen-like hollow elongate outer housing;

(b) an electrically conductive socket at one end of said housing, suitable to receive the outer end of at least one detachable test member capable when contacted with a physiological test liquid of producing an electrical signal correlating with a physiological parameter to which the test member is selectively sensitive and,

(c) a digital read-out window towards the other end of said housing for exhibiting a numerical value corresponding to the parameter.

EP 0 351 892 A2

## Diagnostic Aids and Assemblies Therefor

THIS INVENTION relates to sensor electrodes, and their combination with reference electrodes; to the manufacture of such electrodes; to apparatus utilising such electrodes; and to electrical circuitry into which such electrodes can be incorporated.

Our European Patent Application 82305597 describes the construction of sensors comprising a conductive electrode coated with a mixture, or layers, of a catalytically active enzyme and a mediator compound and usually further coated with a retaining permeable membrane. When such a coated electrode is contacted with a substrate containing a species for which the enzyme exerts a catalytic effect, the mediator compound transfers charge to the electrode and this can be used to give a readout signal, against a standard electrode, correlated with the concentration of the said species, even in the presence of other species since enzymes are typically highly selective in their catalytic action.

Enzymes which have been investigated and utilised in such systems are listed elsewhere in this specification

Thus, numerous types of enzyme-coated electrodes have been utilised each specific to the presence of a physiological or other substrate for which the particular enzyme acts as a catalyst and each therefore potentially capable of acting to detect, measure or monitor the level of the substrate in vivo or in vitro and give a readout correlated for instance with an underlying physiological condition controlling or affecting the substrate level. In particular, use of glucose oxidase or bacterial glucose dehydrogenase as the enzyme, associated with suitable electron-transferring mediator compounds, has been shown to give readout signals correlating linearly with in vitro blood glucose levels over a wide range thus giving a diagnostic or measuring tool for diabetic conditions. Also, such electrodes can measure glucose levels in plasma, serum, interstital fluid, saliva or urine.

The mediator compounds described in our copending Application include polyviologens, fluoranil and chloranil. However, the preferred mediator compounds are metallocene compounds, and in particular the ferrocenes (biscyclopentadienyl iron and its derivatives).

The particular advantages of ferrocenes are as follows:-(a) a wide range of redox potentials accessible through substitution of the cylopentadienyl rings (b) functionalisation of the rings, e.g. to confer solubility or chemical linkability to other such rings or other system components (c) electrochemically reversible one-electron redox properties (d) pH-independent redox potential and (e) slow autooxidation of the reduced form.

The ferrocene structure may be modified by substitution on the rings, and/or by association or polymerisation, which modifications affect the physical, chemical and electrical behaviour so that optimisation of a particular sensor electrode material is possible. In general use, the compound 1,1′dimethylferrocene is a valuable mediator.

Our copending Application of even date herewith entitled "Assay techniques utilising specific binding agents" is concerned with the effect on the enzyme and/or mediator electro chemical availability of specific binding agents e.g. antigens/antibodies and others. It can be embodied by specialised electrodes. Such electrodes fall within the scope of the present invention and are discussed below. The description of the above Application is included herein by way of reference.

The prior art Application referred to above discloses equipment utilising such sensor electrodes. In general it is suitable for research or institutional (e.g. hospital) use. The present invention is concerned with providing sensor electrodes and equipment for use by lay persons or without extensive technical back-up services.

Use may be made of such electrodes in chemical industry especially where complex mixtures are encountered, e.g. in food chemistry or biochemical engineering. They are however of particular value in biological investigation or control techniques, in human or animal medicine.

We have now established certain design criteria in the production of such electrodes for lay, or clinic, use.

The electrodes can be used in an invasive probe( i.e. one which enters body tissue to contact a body fluid such as whole blood or subcutaneous tissue fluid) or as part of an external test upon a withdrawn sample (using a syringe) or upon an expressed sample (e.g. using a needle-prick device). In each instance the electrode must be as small as practical to avoid trauma either on invasion of the tissue or withdrawing of the sample. If invasive, it must be throwaway, to avoid cross-contamination and to prevent encapsulation by fibroblast cells which occurs with long-term implants.

It must be elongate, either to fit within a pointed needle, or for ready handling as an electrode for ready assembly to equipment on the one hand and contact with the sample on the other. It must be sensitively manipulable. It must carry, prior to assembly or in the assembled structure, the reference electrode as well as the 'sensitive' electrode, in spaced non-contiguous relationship.

The present invention, in a major aspect, consists sensor means for selective detection, measurement or monitoring of a given dissolved substrate in a mixture of dissolved substrates, comprising:-

(a) an area of first electrode material comprising an enzyme catalytic of the said substrate and a mediator compound to transfer charge to the electrode when the enzyme is catalytically active, adjacent to but non-contiguous with;

(b) an area of reference electrode material; both electrodes being of small dimension, and extending as or supported on an elongate member to facilitate manipulation before or during contact with live tissue or a small withdrawn sample of body fluid.

The area of first electrode material may comprise (a) a ligand such as an antibody (b) an antiligand, such as a hapten , with specific binding properties thereto (c) the mediator, conjugated to either (a) or (b) and electrochemically active only when (a) and (b) are not specifically bound. Such an electrode is useful in assay of a system which unbinds (a) and (b), at least in part, thereby to provide mediator for the enzyme/substrate reaction.

The sensors can be subdivided for convenience into external sensors, invasive sensors, sensors with dual capacity, and sensors assembled in situ.

A External Sensors

Essentially these are used by dipping into or similarly contacting a liquid substrate e.g. a glucose-containing small blood sample or drop of blood with both electrodes

In one aspect the invention provides a sensor for contact with a liquid mixture of components for detecting the presence of, measuring the amount of and/or monitoring the level of one or more selected components capable of undergoing an enzyme catalysed reaction, the sensor comprising:-

(a) an elongate support member,

(b) on a surface thereof towards one end an expanse of a first electrode of electrically conductive material comprising at least at an outer surface thereof the combination of an enzyme and a mediator compound which transfers electrons to the first electrode when the enzyme is catalytically active,

(c) on a surface of the elongate support member and also towards the said end thereof to be in close proximity therewith an expanse of a second, reference, electrode, and

(d) separate electrical connection to each electrode for attachment to a read-out means denoting presence, amount, or monitored level of the said one or more selected components in a liquid medium into which the support member is dipped to contact both electrodes.

The elongate support could be a rod or tube, but conveniently it comprises a flat strip.

The first electrode is preferably formed of carbon e.g. a filter paper containing carbon We have also found that carbon foil e.g. as available under the Trade Marks "GRAPHOIL" or "PAPYEX" is a valuable electrode material. The enzyme thereon can in theory be any enzyme, e.g. those listed in our European Application itemised above but the use of glucose oxidase or dehydrogenase, e.g. the bacterial glucose dehydrogenase from Acinetobacter calcoaceticus is particularly valuable. Any suitable mediator compound can be used, but ferrocene or ferrocene derivatives (especially 1,1'-dimethylferrocene) are greatly to be preferred.

By way of example only, carbon foil can be glued to the strip; 1,1'-dimethylferrocene mediator can be deposited on the surface of the foil by evaporation of a toluene solution; and enzyme can be bonded to the surface by the use of 1-cyclohexyl-3-(2-morpholinoethyl) carbodiimide metho-p-toluene sulphonate (referred to below as "carbodiimide").

The second electrode can be any convenient reference electrode. We have found it useful to provide adjacent but not contiguous to the first electrode, a flat layer of silver and to convert the surface thereof to silver chloride so as to give an Ag/AgCl reference electrode.

Typically, the electrical connections can be wires which extend down, and are preferably adhered to, the strip, and make electrical contact each with its respective electrode.

The readout means is preferably a digital indicator suitably connected to a dedicated potentiostat which poises the carbon electrode potential at e.g. +150mV Ag/AgCl for a glucose system. The current flowing is then proportional to glucose concentration.

In a particular valuable version of this type of sensor, it comprises (a) a flat first electrode area of known area small enough to be completely coverable by the smear of blood produced from a non-expressed drop of blood generated from a needle-prick at a bodily extremity, (b) a reference electrode area on the same surface separate from but sufficiently close to the sensitive electrode area that the said blood smear also reaches the reference electrode to establish electrical communication and (c) conductive elements extending separately along the same surface of,and thus insulated from the elongate support member, communicating one with each electrode for connection to signal read-out means attachable to one end of the member.

The area of the first (i.e. sensitive) electrode is generally substantially square; it may be rectangular or otherwise shaped, but in any case usually will correspond in area to a square of 5 mm edge length, or below e.g. from 2 to 4 mm.

## B. INVASIVE SENSORS

These are generally needle-like in nature, or fit within a hollow needle.

In one form the invention provides a needle probe electrode carrier for placement through tissue into a measurement location such as a blood vessel, in the form of a generally cylindrical pointed needle having formed therein near the pointed end two flat depressions spaced apart longitudinally, each depression with a floor at right angles to the needle radius and each with a protective shoulder at each end of that floor: wherein the floor of one such depression is coated with an adherent layer comprising an enzyme and a mediator compound to transfer charge from the enzyme when it is contacted with its specific substrate and hence catalytically active; the floor of the other such depression is coated with an adherent reference electrode layer; and separate conductive elements are provided along the surface of the needle, communicating one with each electrode layer, for connection to signal readout means attachable at the outer end of the needle.

As will be apparent from the general disclosure herein, it is envisaged that in one form the sensitive and selective electrode may comprise a glucose hydrogenase (or oxidase) enzyme assocated with ferrocene or a like compound as a mediator compound. The reference electrode can be silver/silver chloride. The shoulders (one to each end of the depression), because of the small overall probe size, protect the electrode coatings as the needle passes through tissue e.g. into a vein.

In yet another form the invention in this aspect provides a sensor for selective detection, determination or monitoring of a given dissolved substrate in a mixture of dissolved substrates, of the type in which an electrode is provided with an enzyme catalytic of said substrate and with a mediator compound to transfer charge to the electrode to provide a read-out signal against a reference electrode: wherein the reference electrode is a metallic needle surrounding, insulated from, and of,or coated with, suitable reference electrode material to, the sensitive electrode. Examples are silver, gold or silver/palladium alloy.

In a special form of this aspect of the invention the sensitive (first) electrode is a carbon fibre carrying a coating including the said enzyme and mediator.

## C Dual capacity sensors

The structure of certain sensors is such that they may be dipped into an extraneous sample, or used within a needle.

One example of such is as sensor for selective detection, determination or monitoring of a given dissolved substrate in a mixture of dissolved substrates, of the type in which an electrode is provided with an enzyme catalytic of said substrate and with a mediator compound to transfer charge to the electrode to provide a read-out signal against a reference electrode: wherein the sensitive electrode and the reference electrode are located on opposite faces of an elongate non-conductive support of rectangular cross-section. In this aspect it is usually envisaged that the cross-section shall be very small, e.g. a square cross-section of less than 0.5 mm edge length, so that the sensor can be located in the bore of the needle. Such a needle/sensor combination is also an aspect of the invention.

In another aspect the invention provides a method of fabrication of sensors of the general type referred to above: in which the sensitive electrodes and the reference electrode are separately fabricated on separable supports and these supports are finally united to provide a sensor. Preferably, again elongate supports of very small thickness are used, for needle-mounting in actual use.

Such electrodes may thus be made of particularly small size. Typically, they can be made to fit within a hollow needle, e.g. a 27 -gauge needle, as already familiar to insulin-injecting diabetic patients. Thus, a permanent or semi-permanent monitor arrangement may be envisaged, since the implanted member is not uncomfortably large.

## D. Assembled sensors

Generally speaking, the present invention envisages sensors made up as a permanent support strip. However, it is also within the ambit of the invention to provide a two-part cell assembly each part containing a elongate recess accommodating one elongate electrode, the first electrode comprising an enzyme and charge-transferring mediator on a conductive support and the second electrode being a reference electrode: the said parts, when assembled with the recesses opposed, defining a throughflow liquid channel between the thereby located and spaced electrodes, whereby the combination is sensitive to the substrate of the enzyme passing through the channel.

The present invention further extends to equipment utilising the above sensors. The equipment can be portable or "desktop".

In one form this aspect of the invention comprises equipment utilising such electrodes to give visible readout correlated with a selected physiological parameter thus being capable of use in human or veterinary medicine by medical or nursing personnel, or by experienced lay subjects on a self-measurement basis.

For convenience, this document will refer hereinafter to blood-glucose-measuring equipment as being typical but not limitative of equipment with which the present invention is concerned.

Diabetic subjects need to measure their glucose levels frequently. Hitherto, a common method carried out by the subject personally is colorimetric test using a blood or urine sample which is applied over a surface area containing a colour-reactive detector chemical, adjacent to a comparison area, to give a colour change which is compared with a chart of colour values as an approximate measure of glucose level.

There are however defects in this method. Firstly, colorimetric changes are quantitatively difficult to assess, especially if the patient has impaired vision as a result of the diabetic condition. Indeed, because of this problem expensive automatic colour comparison equipment may need to be purchased by some subjects for interpreting the test results. Secondly, the blood test, while inherently more accurate than a urine test, needs a large enough sample to cover the test surface. Thirdly, it requires the patient to time the colour development accurately. Since blood samples, on a self-treatment basis are taken from body extremities (fingers, toes, earlobes) they are normally not large enough when obtained by a simple needle-prick, and must in fact be expressed i.e. squeezed or massaged out to form a larger drop. Progressively, the tissue of the extremities becomes scarred and coarsened by such treatment to an extent whereby finding fresh testing sites presents a problem.

In order to embody the invention on a home-diagnostic basis a main object of the present invention in one aspect is as described above the provision of small scale non-traumatic test pieces, either as a small diameter invasive probe electrode or as an external test electrode strip capable of using the naturally-arising small blood droplet from a needle-prick tester, without tissue massage. Examples are described in more detail below.

These small-scale electrodes are intended as single-use throwaway articles and are utilised in conjunction with electrical circuitry and a readout means, to which they must be easily attachable and detachable. Such circuitry and readout means is itself preferably embodied on a very small scale.

We have accordingly found that the totality of the equipment is subject to certain design constraints.

Thus it is a further object of the invention in this form that the device should be nontraumatic to the user either physically e.g. if used with its own invasive probe or psychologically by virtue of its appearance.

It is a further object of the invention in this form that the device should be capable, despite the small size of the throwaway electrode and of the permanent circuitry/readout components, of easy assembly and disassembly even by juvenile or elderly lay users.

It is still a further object of the invention in this form to ensure the relatively expensive permanent circuitry/readout components should, despite their small size, be of a form which minimizes loss or damage.

It is still a further object of the invention in this form to provide a device the display readings of which are visible and understandable to a non-expert user.

We have now found that these and other objects of the invention can be met by assembly the circuitry/readout components into a housing resembling a pen/digital-watch.

According therefore to one aspect of the present invention there is provided an assembly of circuitry and display means for use in producing a readout value as a diagnostic aid in human or veterinary medicine, housed in a pen-like hollow elongate housing having (a) at one end an electrically conductive socket suitable to receive the outer end of at least one detachable test member capable of producing an electrical signal correlating with a physiological parameter to which the test member is selectively sensitive and (b) towards the other end a digital read-out window for exhibiting a numerical value corresponding to the parameter. A thermister may also be used for temperature compensation.

The person skilled in the art of designing medical equipment will appreciate that the invention extends

5

not only to the pen-like assembly as defined above but also to the combination of such an assembly with an attached test member, and to the combination as a kit of interrelated parts of such an assembly with a plurality of test members suitable for one-off use.

The term "pen-like" is a general limitation on size and shape. In functional terms, its characteristics are such that it can be held near the socket between the thumb and the nearer one or two opposed fingers, with the elongate body resting on and extending beyond the forefinger, but not to an extent that prejudices fine control of the socket end by the thumb and fingers. In numerical terms it can be from 10 to 30 cm. long and from 0.5 to 3 cms across its maximum transverse dimension; more usually it will be from 12 to 20 cms. long and from 0.8 to 1.5 cms. across. It can be generally circular, or polygonal, in cross-section. Each detachable test member is usually a small-scale enzyme-coated sensor electrode, of the type discussed in the earlier Patent Applications listed above, and especially such an electrode where the enzyme is specifically glucose-catalyzing whereby diabetic conditions can be measured. It may be a small-calibre invasive probe e.g. based on a 27-gauge needle familiar to diabetics. It may alternatively be a flat external strip electrode dimensioned to operate on a small, non-expressed, blood droplet. The socket arrangement will vary accordingly.

In one embodiment of the present invention, two or more sensor electrodes may be incorporated into a single test member. Again, the socket arrangement will vary accordingly.

The readout means will typically be a conventional seven-segment display window towards the rearward end of the "pen" as in conventional pen/watches. In the case of the multiple sensor embodiment described in the preceding paragraph the display may be switchable between each sensor's discrete monitoring circuit, both the display and a single monitoring circuit may be switchable between sensors, or , a specific display may be provided for each of the sensors present.

Such equipment is of course particularly adapted for use with the non-invasive strip sensor defined above, but may also be used with the needle-type invasive sensors.

Another form of equipment with which the present invention is concerned is so-called "desk-top" equipment, i.e. for general but skilled use in a general clinic.

In this aspect the invention sets out to provide suitable equipment for a practitioner of "desk-top" scale and complexity and while use of such equipment may, according to the invention, be envisaged for any enzyme-catalysable component (with suitable electrode systems) for convenience this specification will refer to glucose determination as typical.

In one form the present invention therefore provides analytical equipment for detecting the presence of, measuring the amount of and/or monitoring the level of one or more selected components of a liquid mixture, comprising:

a cell the parts of which on assembly jointly define a low-volume liquid chamber, at least one of the said cell parts being provided with liquid inlet and outlet ports communicating with the said defined chamber when the parts are assembled;

a selectively operable pump for causing the liquid mixture to flow into the inlet means and out of the outlet means either continuously or intermittently;

a first electrode in the form of a thin expanse of conductive material and comprising at least at an external surface thereof the combination of an enzyme and a mediator compound which transfers electrons to the electrode when the enzyme is catalytically active.

a second, reference, electrode also in the form of a thin expanse of conductive material;

each said electrode presenting an operative surface within said defined low-volume liquid chamber;

a potentiostat for poising a predetermined voltage difference between the electrodes;

means for detecting or measuring current flow between the electrodes when a liquid sample is introduced into the chamber;

and

signal storage and/or readout means operatively connected to the means for measuring current flow.

The cell can be made out of two parts of "PERSPEX" (Trade Mark) rigid polymethylmethacrylate, and bolted together with the intermediary of a silicon rubber spacing gasket. It can possess two abutting faces, one or both recessed if and as necessary to define a suitable chamber, which is usually elongate in the direction of liquid flow, and the volume of which can vary, for example, from 0.1 to 1.0 ml. This will cope with a typical glucose-containing liquid sample in about one minute continuous flow to an accuracy of ± 3%. The liquid inlet and outlet ports can both be in one cell part, or one in each.

The component to be measured is preferably glucose so as to give a"desk-top"glucose analyser for the practitioner. The enzyme used is therefore preferably glucose oxidase (it can be a glucose dehydrogenase) and for this ferrocene or a ferrocene derivative such as 1,1'-dimethyl ferrocene is a preferred mediator compound.

The support electrode is preferably of carbon or a carbon-containing mixture . The carbon foils and meshes known under the Trade Marks "GRAPHOIL" or "PAPYEX" have been used in practice with good results.

The other electrode is preferably of silver foil with an electrodeposited AgCl layer.

Strip electrodes as defined can lie one on each opposed face of the defined recess.

The pump will generally be operated continuously throughout a determination since the glucose in the cell is catalysed and the reading therefore decreases. If desired the pump can itself operate an injector means dosing or metering the flow of liquid.

The potentionstat can for example be such as to hold the carbon electrode at +150 mV vs Ag/AgCl. The current flow will be proportional to glucose concentration over all expected ranges.

In the operation of a glucose sensor a number of relevant technical points and advantages should be considered. It will be appreciated that these points are of general relevance and should by no means be restricted in this application to the particular design features of the present application: in other words, they apply generally to the embodiments listed above, and especially to those embodiments insofar as they deal with equipment and methods for glucose sensing. These features are:-

## I Constructional Features

### (a) Membrane cover for electrode

Although the enzyme electrode should be in electrical contact with the liquid, it may be found valuable to exclude the sensor from interfering contact with larger moledules or tissue fluid components. This can be done by a covering or surrounding membrane, depending on electrode geometry. Heat-shrinkable thin polymer tubing can be used as, or in connection with, such membranes.

The membranes can be polymerised in situ (e.g. cellulose acetate). A particular valuable membrane is formed by polycarbonate, especially those polycarbonates sold under the Trade Marks "NUCLEOPORE" or "STERILIN". When tissue fluids are examined they may contain ascorbate; polycarbonate membranes do not permit the passage of ascorbate and thus virtually eliminate interference from that substance. Alternatively a polyurethane membrane may be employed.

### (b) Type of carbon

Carbon foil, as strips, or carbon attached to metal meshes, of pyrolytic grade and known by the Trade Marks "GRAPHOIL" and "PAPYEX" are much preferred for carbon-ferrocene electrodes for use with glucose oxidase. Oxygen interference is minimal, there being less than 4% change in signal between anaerobic and fully aerobic samples. Their physical nature is also very convenient for fabrication, especially of small-scale devices.

## II Operational features

### (a) Operational potential

Preferably operation should take place at a potential equivalent to +50 to +200 M V vs. SCE since interference caused by oxidation of other chemical species present is thereby reduced.

### (b) Concentration range

Glucose oxidase can be used to monitor glucose concentrations of 0 to 40 mM, and glucose dehydrogenase at 0 to 20 mM when immobilised on a carbon-ferrocene electrode. The sensor response is linear up to about 40 mM.

### (c) Response times

The glucose oxidase sensor without membrane is kinetically limited giving rapid response times i.e. about 20 seconds to 95% of the steady-state current response.

### (d) Oxygen-sensitivity

Glucose dehydrogenase/ferrocene electrodes are totally oxygen-insensitive.

### (e) Use of third electrode

In practice, a realistic device can achieve good performance without a third electrode, using Ag/AgCl as a reference counterelectrode, as described more fully below.

### (f) pH and temperature

Glucose oxidase electrodes show no change in current output between pH6 and pH9, and are thus relatively pH-insensitive. They are temperature-stable up to 40°C. If necessary temperature compensation can be effected using a thermistor, or a constant temperature jacket may be used. Also, operating with the electrodes diffusion-limited minimises temperature effects.

### (g) Storage of Electrodes

Electrodes may be stored moist. Extended storage, over months or years, may be achieved by freeze-drying or airdrying.

Although the invention as defined above has been discussed in terms of the equipment used, it will be appreciated that other aspects of the invention also present themselves. The totality of the equipment may include a replaceable or throwaway cell; thus, the cell per se as defined above is an aspect of the invention, as are the combination of electrodes irrespective of details of cell design, and the individual electrodes, of novel configuration. Methods of detecting the presence of, measuring the amount of or monitoring the level of one or more desired components (e.g. glucose) of a liquid mixture (e.g. tissue fluid or liquids derived therefrom) utilising the equipment cells or electrodes cells defined above are also a feature of the present invention.

Finally, the present invention is concerned with the electrical circuitry for operating the equipment as described.

According to this aspect of the present invention there is provided a measuring device for use with an electron-transfer electrode, comprising means for comparing an electrical output of the electrode with an electronic reference and means for providing a signal related to the electrical output of the electrode.

By employing an electronic reference rather than a cell or reference electrode a measurement using a sensor including an electron-transfer electrode may be made without the use of a separate electrode as a reference.

In a preferred embodiment of the invention, the electron-transfer electrode is poised at a fixed potential against a reference electrode, and the current flowing in the electron-transfer electrode is measured.

The particular electron transfer electrode may be selected from a range of electrodes including those employing the following enzymes

| Enzyme | Substrate |
|---|---|
| **Flavo-proteins** | |
| Pyruvate Oxidase | Pyruvate |
| L-Amino Acid Oxidase | L-Amino Acids |
| Aldehyde Oxidase | Aldehydes |
| Xanthine Oxidase | Xanthines |
| Glucose Oxidase | Glucose |
| Glycollate Oxidase | Glycollate |
| Sarcosine Oxidase | Sarcosine |
| Lactate Oxidase | Lactate |
| Glutathione Reductase | NAD(P)H |
| Lipoamide Dehydrogenase | NADH |
| | |
| **PQQ Enzymes** | |
| Glucose Dehydrogenase | Glucose |
| Methanol Dehydrogenase | Methanol and other Alkanols |
| Methylamine Dehydrogenase | Methylamine |

Haem-Containing Enzymes

| | |
|---|---|
| Lactate Dehydrogenase | Lactate |
| (Yeast Cytochrome B2) | |
| Horse-radish Peroxidase | Hydrogen Peroxide |
| Yeast Cytochrome C | |
| Peroxidase | Hydrogen Peroxide |

Metalloflavoproteins

| | |
|---|---|
| Carbon monoxide | Carbon Monoxide |
| Oxidoreductase | |

Cuproproteins

| | |
|---|---|
| Galactose Oxidase | Galactose |

The invention will be further described with reference to the accompanying drawings, in which:-

Figure 1 is a front view of a strip-supported electrode configuration;

Figure 2 is a back view of the combination shown in Figure 1;

Figure 3 shows an invasive probe electrode;

Figure 4 shows an alternative strip-supported electrode;

Figure 5 shows a strip-supported electrode which is a variant of Figure 4;

Figure 6 shows a modified connection of the strip electrode of Figures 4 and 5;

Figure 7 shows a further alternative supported electrode;

Figure 8 shows a combination of two electrode supports;

Figure 9 shows one of the component parts of Figure 8 lcated within a needle acting as a reference electrode,

Figure 10 shows a carbon fibre treated to form an electrode and located as in Figure 9;

Figure 11 shows an assembly of two coordinated elongate electrodes held in spaced relationship but capable of separate replacement;

Figure 12 shows diagrammtically the electrical performance of electrodes as described in the above Figures, with especial reference to Figures 7 to 10;

Figures 13a and l3b are general diagrammatic side views of a pen-like portable holder, of particular utility for the electrodes shown in Figures 4, 5 and 6, having an assembly of circuitry and having a read-out window;

Figure 14 is a general diagram of "desk-top" analytical equipment for measuring a substrate such as glucose in a liquid sample, utilising the electrode assembly of Figure 11;

Figure 15 is an exploded view of part of the equipment of Figure 14;

Figure 16 shows a schematic diagram of one form of electrical circuitry for use with the electrodes and equipment of the present invention;

Figure 17 shows a more elaborated circuit diagram for use in the embodiment of Figure 16;

Figure 18 shows a schematic diagram of an alternative embodiment of electrical circuitry; and

Figure 19 shows the more elaborated circuit diagram of a yet further embodiment of circuitry.

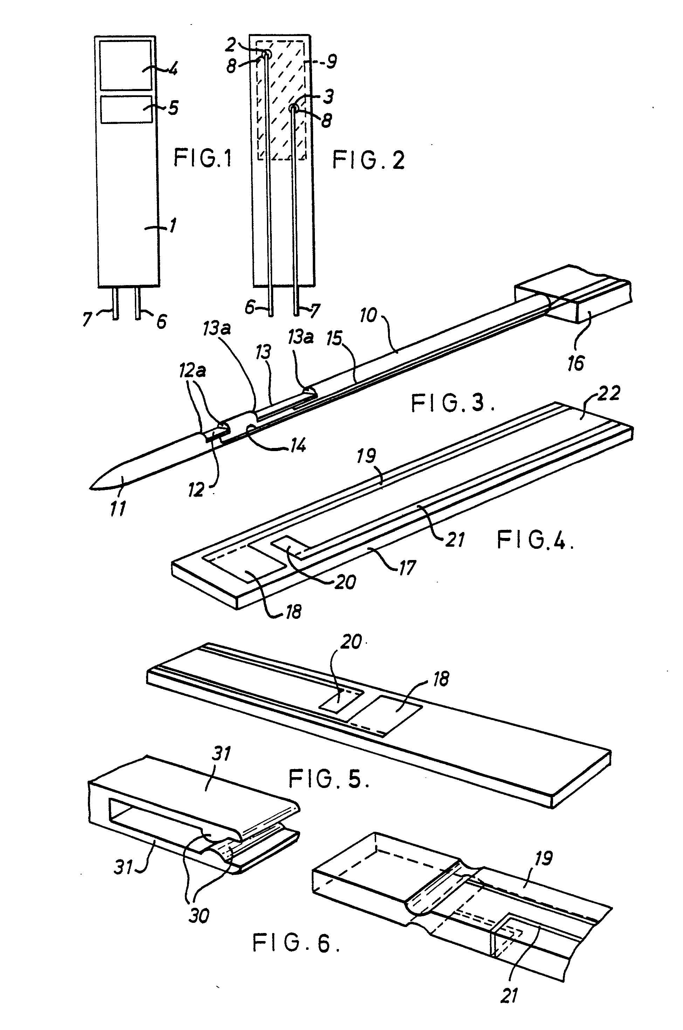

In the following description of Figures 1 and 2 dimensions, materials amount and proportions are given by way of example only.

A strip of epoxy glass 1, 9.5 x 40 x 1. 6 mm, has two 1 mm diameter holes 2 and 3 therein. A 9 x 9 mm piece of graphite tape or foil 4 is glued on one face, near the end to cover hole 2 and a 4 x 9 mm strip of silver foil 5 is glued adjacent thereto over hole 3. Wires 6 and 7 (Fig. 2) on the back enter holes 2 and 3 respectively for electrical connection with the respective electrode material 4 and 5, being glued in the holes by conductive epoxy resin 8. A stabilising layer of epoxy resin is present over at least part of the back e.g. at 9 to keep the wires in place. Carbon electrode 4 is covered with 1, 1'-dimethylferrocene and glucose oxidase. Silver electrode 5 is covered with silver chloride.

The strip is made up in the following sequence:-

(a) drill holes 2 and 3,

(b) glue on electrodes 4 and 5; "ARALDITE" epoxy resin is suitable but should not enter holes 2 and 3,

(c) attached wires 6 and 7, using conductive epoxy 8, and apply "ARALDITE" resin at 9 the fix the wires in place,

(d) hold the silver electrode at +400 mV vs. SCE in 5M for 10-15 seconds to deposit a thin AgCl layer,

(e) apply a solution of 1,1'-dimethylferrocene (4 l) in toluene (20 mg/m ) to the graphite tape 4 and allow to evaporate,

(f) cover the ferrocene-coated tape with 50 of carbodiimide (25 mg/ml) in pH 4.5 acetate buffer for 1 1/2 hours and

(g) rinse and cover with glucose oxidase (12.5 mg/ml) in pH 5.5 acetate buffer for 2 hours.

The strip can be used by attaching wires 6 and 7 to a potentiostat poising the potential at electrode 4 at +150 mV. vs. Ag/AgCl, and dipping the strip into a glucose-containing solution so that both electrodes 4 and 5 are covered. The shape of the strip facilitates such handling. The current flowing is proportional to glucose concentration.

Figure 3 shows a 27-gauge (0.3 mm) needle 10 pointed out at 11 and having a smaller area 12 and a larger area 13 cut from its curved surface as shown and insulated from the body of the needle . Area 12 is coated with silver to provide a reference silver-siver chloride potential. Area 13 is coated first with carbon and thereafter with a mediator compound such as ferrocene and with a glucose-sensitive enzyme e.g. bacterial glucose dehydrogenase. A protective membrane can if desired also be located over this deposit. It is to be observed that the recess area 13 is a well-defined area of accurate size; also that the shoulders 12a and 13a protect the respective deposits located between them as the needle is passed through tissue.

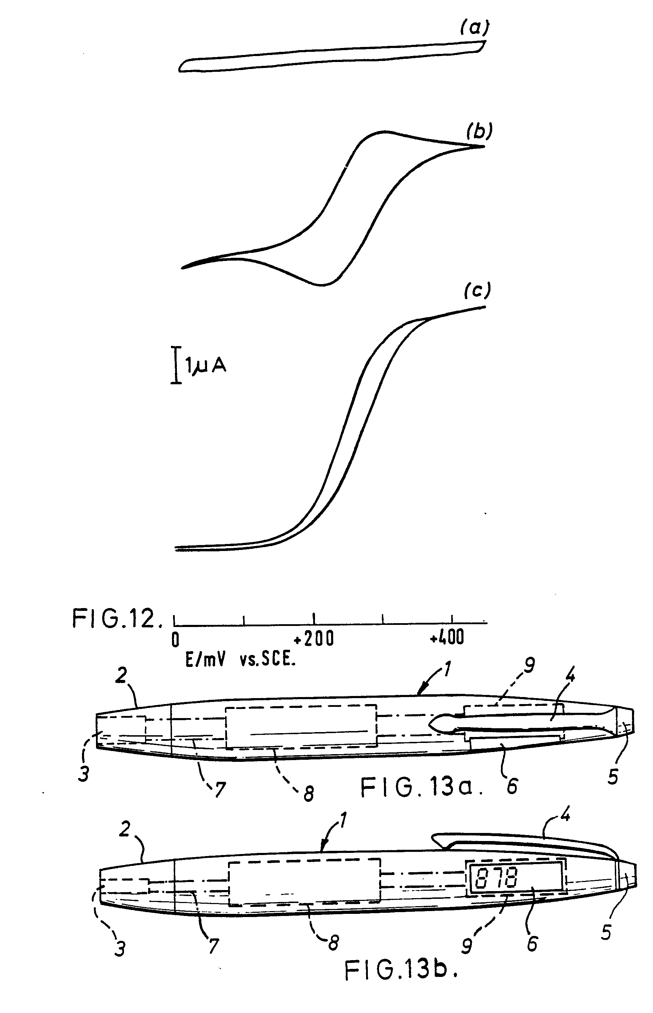

Deposited conductive lines 14 and 15 pass separately along needle 10 and over its shaped end 16, the exact path of these lines not being of major significance except that they should not touch and that they should only connect one way round if head 16 lies in a connecting socket cavity as described in more detail below with reference to Figures 13a and 13b. Conductive lines 14 and 15 are insulated from the body of the needle

Figure 4 shows a strip electrode 17 made of, for example, a ceramic material or printed-circuit-board laminate. It includes a square area 18 with connector lead 19, the square being covered with the enzyme-containing layers as described above. It further includes a small reference electrode area 20 and separate connector lead 21. The rearward end 22 of the electrode 17 fits into a socket as shown in Figures 13s and 13b and described below. It is to be noted that, as with the needle 10, the electrode strip 17 is a small-scale device. Thus square area 18 is of a side length only about half that of each of two swuare colourimetric test areas of conventional diagnostic tests and can be used with the original non-expressed bead of blood from a needle-prick device, which is adequate to cover the whole of the square area and communicates electrically with reference electrode area 20.

Modifications may be made to the embodiments shown in Figures 3 and 4. For example the strip electrode as shown in Figure 4 can be longer, whereby electrodes 15 and 20 are located only partway along the strip, leaving a free end 22a to facilitate ease of handling by the patient without damaging or touching the electrodes. Also, the electrode strip can clip within two opposed contacts or resilient mounting 31, the routing of one or other of conductive lines 19 and 21 being modified accordingly.

Figure 5 shows a longer strip, and Figure 6 shows the inner end of a strip held between two resilient metal contact strips.

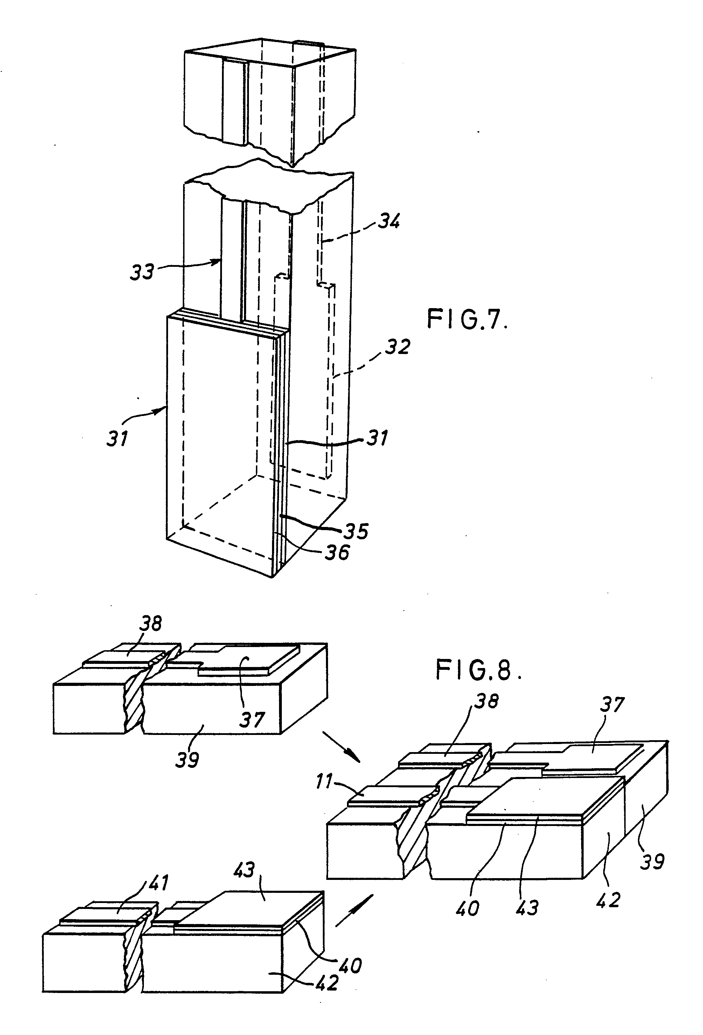

In Figure 7 a 2 cm length of electrically insulating polymer for example MYLAR or TEFLON ( a polyfluorocarbon) 0.3 mm square in transverse cross-section is provided with a palladium-silver conductive electrode 31, on the front surface as shown, and a second, smaller electrode 32, on the back as shown in dotted lines. In each case conductive lines 33 and 34 respectively, were formed simultaneously with the electrodes.

On the front electrode 31 is painted a mixture of toluene, 1,1'-dimethyl ferrocene and graphite, formed by mixing a solution of the toluene and 1,1'-dimethylferrocene and a slurry of toluene and graphite. It is believed that the ferrocene is adsorbed on to graphite particles. After drying the mixture forms a layer 35. A layer 36 of glucose oxidase is then immobilised on the graphite surface by carbondiimide immobilisation, known per se (enzyme adsorption can also be used). The electrode may then be covered, on both sides, with a semipermeable membrane of cellulose acetate (or polyurethane), not shown, to block large interfering species from contact with the electrode.

The square section of the support helps in the painting of slurry, or the enzyme-attachment stages, in keeping the electrodes 31 and 32 distinct.

The small scale electrode so produced could be used per se but is especially valuable for incorporation into a standard gauge needle, giving a blood-glucose reading using the same invasive member as the eventual injection.

When producing such small scale needles, the exact sequence of steps can vary. For example, graphite could first be painted on, and a solution of the mediator (ferrocene, etc) in toluene then be applied by dipping into the graphite. Likewise, the enzyme can be applied in solution for absorption. There is therefore a danger that the reference electrode e.g. silver/palladium could be adversely affected by the solvents or solutes used.

Figure 8 shows the key steps of a procedure which can be used to advantage in the fabrication of these microelectrodes.

The reference electrode 37 and its conductive lead-out strips 38 are formed in silver/palladium on TEFLON base 39. Similarly, an electrode support 40 and lead-out strip 41 are formed in silver/palladium on TEFLON base 42. Only this base 42 and its electrode support are then subjected to (a) painting on a graphite slurry in toluene (b) dipping in 1,1' dimethylferrocene solution in toluene and (c) contacting with the enzyme to absorb e.g. glucose oxidase into the active layer 43. Thereafter the bases 39 and 42 are glued or held, side-by-side, their general rectangular cross-section facilitating such positive location. Back-to back location is also possible.

As before, the finished assembly may be located inside a needle bore, e.g. with extra access portions near the electrode surfaces.

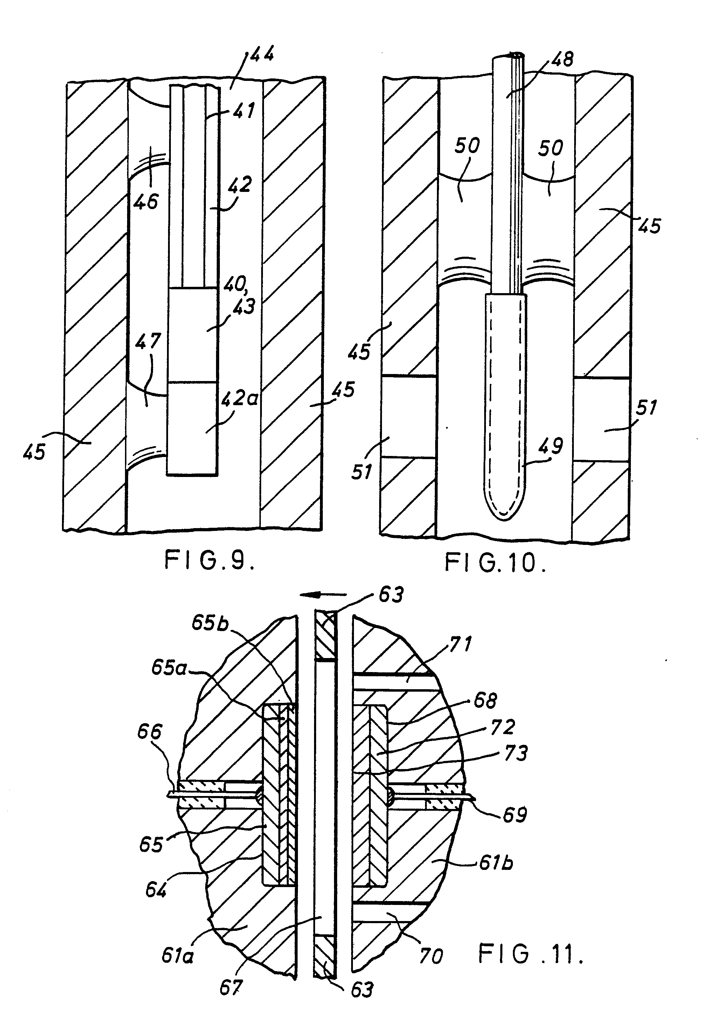

Separation of electrodes in this way leads to a further proposal, as shown in Figure 9, in which the base 42 and its adherent conductive support 40 and active layer 43 are located in an electrically insulated fashion within the bore 44 of a needle 45 by insulating adhesive at 46 and 47. It will be found convenient to allow som uncovered free space 42a at the end of base 42 to facilitate such attachments. Any insulating fixing which does not affect liquid flow could equally well be used.

Needle 45 is this embodiment must be made of or coated with a suitable reference material e.g. silver/palladium, or gold, or silver, since it acts as a reference electrode. A silver coating may be electrochemically plated with chloride ions to make an Ag/AgCl reference electrode

The electrode need not be fixed longitudinally with respect to the needle; it could lie within the needle during insertion and, by partial withdrawal of the needle, becomes exposed to e.g. interstitial fluid at the (projecting) electrode region.

Typically, the wire will be 50 m in diameter. It can be covered by a dip coating procedure e.g.

I. Dip-coated wire with a collidal suspension of colloidal carbon, a ferrocene and glucose oxidase in buffer in a mixture of organic solvents (such as acetone).

II. Dip-coat the wire with a membrane such as cellulose acetate, polyurethane or other suitable solvent.

Alternatively, the carbon material may be dispensed with entirely and ferrocene bound directly to the gold surface. Suitable ferrocenes for this procedure are thiol-substituted. A matrix of cross-linked glucose oxidase may then be formed by dipping the electrode in a solution of glucose oxidase in buffer and glutanyaldehyde and proceeding as in II.

Figure 10 shows a further development of this idea. Carbon fibre 48 is (a) dipped into a toluene solution of ferrocene (b) dipped into a solution to immobilise glucose oxidase thereon using carbodiimide and (c) dipped again in cellulose acetate, at 49. Polyurethane membranes, or enzyme adsorption could also be used.

Again, as in Figure 9, the fibre can be mounted in a reference electrode needle 45 e.g. on insulating supports 50, possibly using suitable ports 51 as blood access ports.

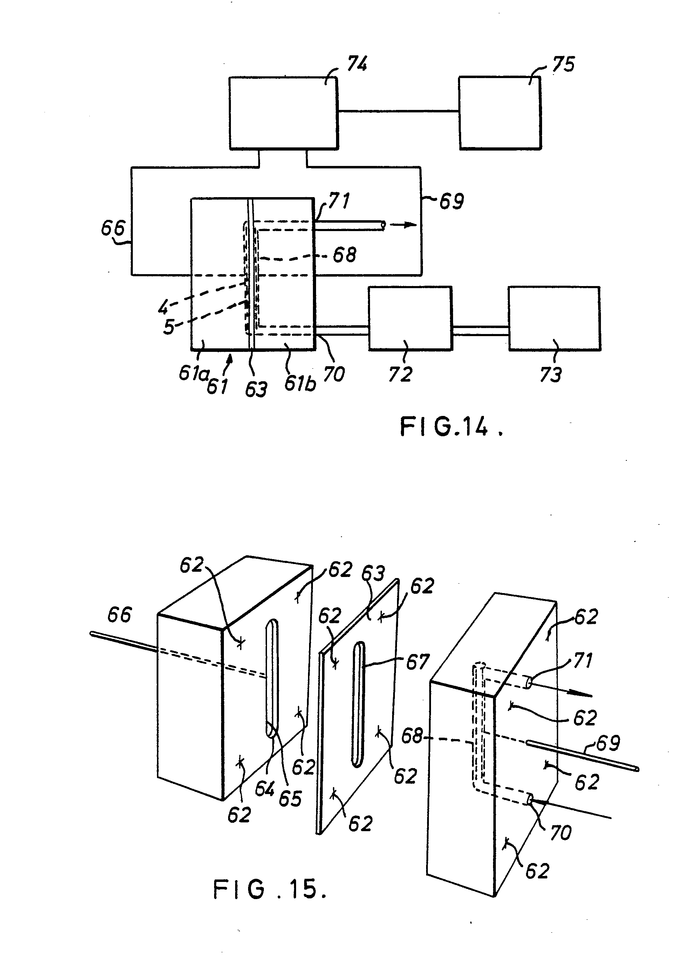

Figure 11 shows two halves of a throughflow cell described in more detail below with reference to Figure 15, the reference to apparatus feature being the same as utilised therein and described more fully below. The cell has two like elongate shallow recesses 64 and 68 one on each half 61a and 61b respectively which with spacer 63 define a working chamber when the cell is assembled.

Within recess 64 is a strip 65 of carbon as sold under the Registered Trade Mark GRAPHOIL, coated at 65a with a layer of 1,1'-dimethylferrocene deposited from toluene solution and at 65b with the enzyme glucose oxidase bonded by carbodiimide. The recess 64 holds strip 65 in opposed relation to a strip of silver foil 72 with a surface silver chloride layer 73, itself located and held in recess 68.Thus, a definite relative electrode configuration is achieved with miniature elongate sensor electrodes even though the "active" and "reference" electrodes are located on different, separate,parts.

Further description of the cell 61 and its environment in use is given below with reference to Figure 14 and 15.

Figure 12 shows results obtained when the 0.3 sq mm prototype electrode of Figure 7 was characterised electrochemically and is generally indicative of results obtained with other electrode designs.

First, a solution of graphite powder plus a binder in toluene was painted on the palladium-silver alloy conductor 31(supplied by Ferranti) and allowed to dry. Curve (a), shows a direct current cyclic voltammogran of the electrode in 100mM phosphate-perchlorate electrolyte at pH 7.0. The electrode displays a potential window over the range -300 to +500 mV vs SCE. This behaviour is similar to that of a conventional graphite electrode. The electrode surface was mechanically sound and could be polished with an alumina-water slurry without affecting its response.

## Reference electrode

The reference potential of the palladium-silver reference electrode 32 (incorporated on to the strip) was determined by substitution into an electrochemical system which had previously been calibrated with a saturated calomel electrode (SCE). The reference potential was 60 mV negative of SCE, consequently the glucose sensor should be operated at 160+200mV vs Ag/Pd. The reference potential was stable, i.e. did not drift, over 48 hours of operation.

Curve (b), shows a direct current voltammogram of ferrocene monocarboxylic acid recorded at the graphite electrode.

Curve (c) shows that electro-oxidation of glucose occurs upon addition of glucose and glucose oxidase to the solution. Together, these curves demonstrate that the electrode formed the basis of a glucose sensing device.

## Incorporation of 1,1'-dimethylferrocene into the electrode

A solution of 1,1'dimethylferrocene in toluene was mixed into a tolune-based slurry of the graphite powder. The mixture was then painted on to the base conductor 31 and allowed to dry at 35. This provided an electrode surface that was electro active towards glucose oxidase.

## Conclusions

These experiments showed that "thick layer"or screen-printing technology could provide a usable base strip which could easily be coated with a stable graphite surface and that moreover the electrode surface could be made of electro-active towards glucose by adsorption of a ferrocene directly into the coating mixture. In addition, the reference electrode operated satisfactorily in buffered solutions.

Figures 13a and 13b show a holder which is particularly adapted to utilise electrodes as shown in Figures 4, 5, 6 but which could if necessary utilise electrodes as shown in Figures 1 and 2, 3, and 7 and 8 at least of the various embodiments shown.

From above the holder 81 intentionally resembles a conventional pen/watch as much as possible. It has a forward end 82, possibly rotary to tighten the walls of a flattened socket cavity 83 formed within it. A central join, a clip 84 and a press-button 85 all resemble those of a conventional pen, and digital readout-window 86 is also of a type known in pen/watches.

Inside the holder as shown by dotted lines is connection circuitry 87, possibly printed in situ, battery 88 and operating circuitry 89 behind and manufactured as a unit with the display window 86. The display can be capable of operation only when button 85 is pressed so that extra illumination can be provided if necessary.

The embodiments shown in Figures 13a and 13b especially when used in conjunction with the electrodes of Figures 4 - 6 fulfill the design criteria discussed above for such portable equipment.

The delicate manipulation facilitated by the pengrip (e.g. by thumb and finger) means that the small electrodes e.g. of Figures 4, 5 or 6 can be easily assembled into, or detached from, the socket. A user will always orient the holder with the window 86 visible thus always giving a uniform relative orientation to the socket 83 whereby the rearward ends of the fragile electrodes can be fitted without experiment and damage.

The "pen" format is instinctively picked up after use and safely carried in a pocket, more so than for any other small device. Thus the expensive part of the equipment is safeguarded. Furthermore, it is possible to incorporate a conventional timer circuit into the device thereby fulfilling the actual function of a pen-type watch and providing an audible or visible signal which marks the point in time at which a reading should be taken.

Finally, the display is numerical, clearly visible and if necessary can be supplemented by an illuminating light source.

Figures 14 and 15 are closely related to Figure 11, which shows an electrode configuration. A two part cell 61 has respective halves 61a and 61b bolted together by bolts not shown passing, for example through the corner regions where indicated at 62. The parts 61a and 61b are spaced by a silicone rubber spacer 63.

Part 61b has an elongate shallow recess 64 on an internal face, at the base of which is located strip 65 GRAPHOIL carbon foil coated on its outwardly facing surface with 1,1'dimethylferrocene (deposited from toluene solution) and also having bonded thereto by carbodiimide the enzyme glucose oxidase. The midpoint of the rear face of this strip is connected to wire 66 passing sealingly through the cell part 61a.

The spacer 63 has a central elongate hole 67, matching in its periphery the periphery of recess 64.

The part 1b has a shallow elongate recess 68, also of matching periphery to hole 7. At the base of this recess is located a silver foil electrode 72 on which a thin silver chloride layer 73 has been electrodeposited. This is connected at the midpoint of its rear face to wire 69 again passing sealingly through cell part 61b.

Part 61b further possesses a through port 70, communicating with the bottom of the recess, and a through port 71 communicating with the top.

The cell is assembled by bolts 62 so that the recesses 64 and 68 together with hole 67 of spacer 63, define a leak-proof chamber with opposed electrodes. Hydraulically, it is connected at 70 to injector device 72 (e.g. a sample-holding three-way valve) pressurised by pump 73 whereby liquid sample is forced in at 70, through the chamber 64, 67 and 68, and out to waste at 71. Electrically, wires 66 and 69 are connected to potentiostat 74 to poise the graphite electrode at +150 mV vs Ag/AgCl, and current values proportional to glucose content in the sample are displayed at 75.

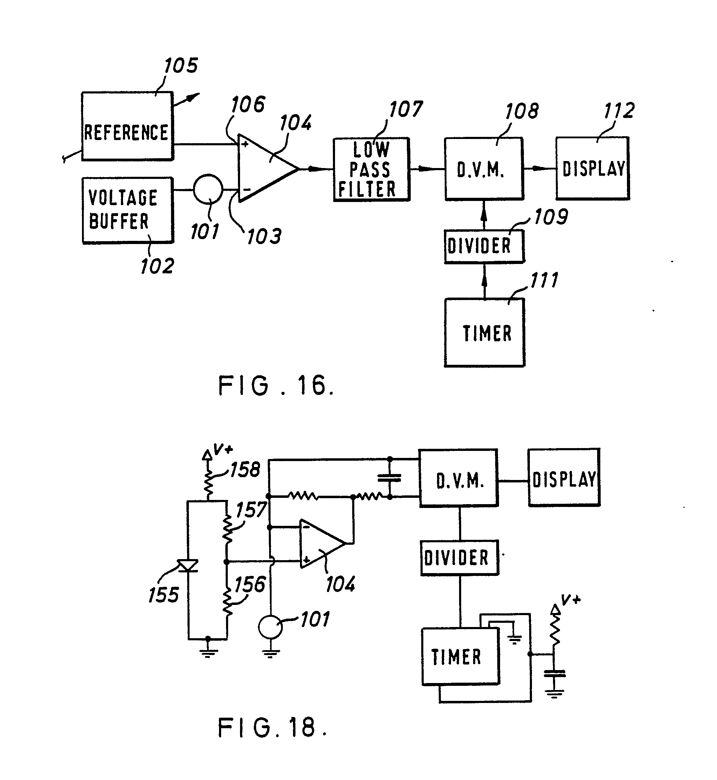

In Figure 16, a sensor 101 is connected between a voltage buffer 102, and the inverting input 103 of the operational amplifier 104 which is configured as a current amplifer. An electronic reference 105 connected to the non-inverting input 106 of the operational amplifier is fed into a low-pass filter 107 which removes rapid signal fluctuations (which may be due to noise, earth hum or other sources of interference) while allowing the filtered output of the operational amplifier 104 to be fed into the digital volt meter (D.V.M.).

The digital volt meter is supplied with clock pulses via the divider 109, from the timer 111. The D.V.M drives a liquid crystal display 112. The electronic reference 105, is either of a pre-selected value or capable of being selected for a particular electron transfer electrode.

In each of the embodiments of electrode discussed above the sensor comprises a mediator-carrying surface which has one or more enzymes immobilized thereupon. The sensor further includes a silver/silver chloride (Ag/AgCl) internal reference electrode. If, for example, a voltage of +200 mV volts is preferentially dropped across the electrode as is the case with a glucose-oxidase-containing electrode, then the reference voltage 105 is selected accordingly.

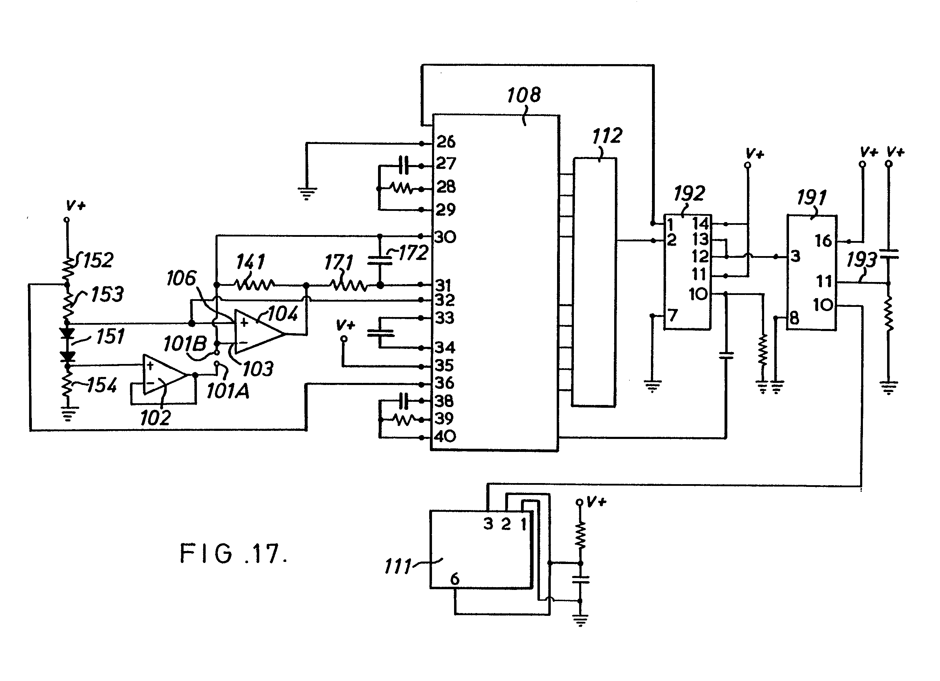

In Figure 17, the electronic reference comprises resistors 152, 153 and 154 together with diodes 151. A voltage is selected at 0.3V by a suitable choice of resistor values at 152, 153 and 154. The voltage across the resistor 153 is 1V, which ensures that 100 A of electrode current will give a full scale reading of 999 on the liquid crystal display 109.

Non-inverting buffer 102 ensures that the voltage on the terminal 101A remains substantially constant The sensor 1 (Figure 1) is connected across terminals 101A and 101B.

The operational amplifier 104 is connected via its inverting input 103 to the terminal 101B and the feedback resistor 141. The non-inverting input 106 is connected to the electronic reference.

A first-order low-pass filter 171, 172 is connected across the feed-back resistor 141, and supplies an analog signal to the D.V.M. 8. Pin values are given for a 7116CPL chip (manufactured by Motorola). The D.V.M. drives a liquid crystal display 112.

Clock pulses for the D.V.M. are supplied from the timer 111, (pin values are given for a 555 chip) via the dividers 191 (pin values for an MC 14020B) and 192 (pin values for an MC14016B chip). The connection 193 to pin 11 of the divider 191 enables a power-up reset.

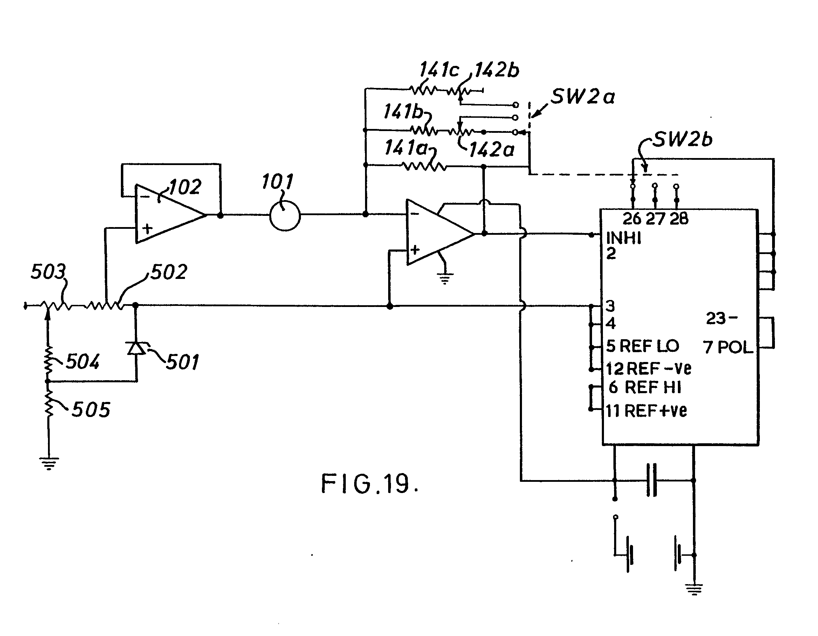

In Figure 18 alternative circuit is shown which does not make use of the non-inverting voltage buffer 102, but has the sensor 101 connected between the operational amplifier 104, and ground. The embodiment shown in Figure 18 employs a fixed reference voltage which is provided by a circuit differing from that

14

In this embodiment, the diode 155 functions as a voltage-reference diode and provides a reference voltage drop across its ends equal to the diode forward voltage.

By a suitable choice of the values of the resistors 156, 157 and 158 the correct voltage may be applied across the electrode. In this embodiment the sensor again employs as a reference an Ag/AgCl couple and immobilized glucose oxidase in the presence of a mediator compound as the electron-transfer electrode.

Figure 19 shows a third embodiment of the present invention, which provides a continuously variable reference voltage which may be selected to accommodate any type of electron-transfer electrode, that is, one, which for example, employs any of the enzymes listed herein or any combination of these enzymes. The LED display is not shown in Figure 4.

In Figure 19 the feedback resistor 141 in circuit at any given time may be selected from resistors 141a, 141b, and 141c by means of switch SW2a which is ganged with switch SW2b. This allows the current output of the sensor 101 to be displayed in three ranges, for example, 1 A, 10 A or 100 A full scale. Furthermore, the range may be trimmed by using the variable resistors 42a and 42b.

The embodiment shown in Figure 19 has the non-inverting voltage buffer 102 of the embodiment shown in Figure 17.

The reference voltage for the embodiment shown in Figure 19, is derived from the circuit elements 501-505 which include the potentiometers 503 and 502 providing a variable voltage across the sensor 101, thereby accommodating any type of electron-transfer electrode. It is envisaged that the continuously variable resistors 142a and 142b could be replaced in certain applications by stepwise resistance switching means with each position or setting being dedicated to a particular type of electrode.

Various modifications may be made in the circuitry. For example the liquid crystal display may be replaced by a plotter or a dosage control device, or a temperature stability circuit may be incorporated.

Various modifications may also be made in constructional techniques for the electrode manufacture.

The electrode may for example be manufactured by screen printing techniques e.g. in a multi-stepped procedure comprising:-

I - screen printing of Ag/AgCl reference electrode and metal tracing.

II - screen printing of the active electrode with a printing ink comprising a colloidal carbon, glucose oxidase in buffer, and an organic polymer.

III - screen printing, spraying or dip coating to provide a membrane over the assembly.

Advantages of this method are that it is amenable to high volume automation, and is of high reproducibility

From the above it follows that a suspension in a liquid medium of carbon together with at least one of (a) an enzyme and (b) a mediator compound capable of transferring charge to the said carbon from the enzyme when the enzyme is catalytically active, the said suspension being formed as a printable and conductive ink for use in the fabrication of electrodes as described above, is also an aspect of the invention. Preferably, both the enzyme (e.g. glucose oxidase) and the mediator (e.g. ferrocene or a ferrocene derivative) are present in the ink formulation.

The mediator may be in the form of a mediator/hapten conjugate, i.e. be linked to a ligand material so that its activity in its charge-transferring property is a measure of further or competitive binding reaction with a specific binding agent with which the eventual electrode, having the specialised ink thereon, is contacted. A specific example is the theophylline/ferrocene conjugate described in copending Application entitled "Assay systems utilising specific binding agents", of even date herewith. Other mediator/enzyme/ligand systems can also be utilised in the specialised ink.

That copending Application also describes a further modification of electrode treatment in which a metal such as gold is pretreated with a mediator compound such as a ferrocene containing a sulphur-containing linking group. Examples of preparation of such materials as the direct monothiol, the low alkylene - omega monothiols ($C_1$-$C_6$) the compound $FC-CH_2-CH_2-CH_2-CH_2-CH_2SH$ or the compounds in which a chain of sulphur atoms connect the ferrocene ring systems are given in that copending Application, the disclosure of which is incorporated herein by way of reference.

Thus, an electrode dip or contact solution for a noble metal electrode such as gold can be prepared containing such a sulphur-linkable ferrocene, and give a coated electrode with a ferrocene-type layer adherently in place.

**Claims**

1. An assembly of circuitry and display means for use in producing a readout value as a diagnostic aid

15

in medicine, and comprising:

(a) a pen-like hollow elongate outer housing;

(b) an electrically conductive socket at one end of said housing, suitable to receive the outer end of at least one detachable test member capable when contacted with a physiological test liquid of producing an electrical signal correlating with a physiological parameter to which the test member is selectively sensitive and,

(c) a digital read-out window towards the other end of said housing for exhibiting a numerical value corresponding to the parameter.

2. An assembly as claimed in claim 1 from 12 to 20 centimetres long and from 0.8 to 1.5 centimetres across its maximum transverse dimension.

3. An assembly as claimed in claim 1 in further combination with an invasive needle probe as the test member.

4. An assembly as claimed in claim 1 in further combination with a flat strip electrode as an external non-invasive test member for receiving a blood droplet.

5. The operative combination of (I) an assembly of circuitry and display means for use in producing a readout value of blood glucose level as a diagnostic aid in treatment or control of diabetes, comprising:

(a) a pen-like hollow elongate housing;

(b) an electrically conductive socket at one end of said housing suitable to receive a detachable strip electrode carrier, and

(c) a digital readout window towards the other end of said housing connected to said socket for exhibiting a numerical value corresponding to the said glucose level and

(II) a strip test electrode carrier held at one end in said socket.

6. The operative combination of claim 5, wherein the strip test electrode carrier is carrying:

(a) a flat electrode of area not greater than 25 sq. mm and comprising a glucose utilising enzyme and a metallocene mediator whereby it is sensitive to blood glucose levels,

(b) a reference electrode area non-contiguous with but closely adjacent to the said sensitive electrode area and

(c) separate conductive elements extending along the strip communicating one with each electrode for connection to the socket.

7. A measuring device for use with an electron-transfer electrode, comprising means for comparing an electrical output fo the electrode with an electronic reference and means for providing a signal related to the electrical output of the electrode.

8. A measuring device as claimed in claim 7 in which the electron-transfer electrode is poised at a fixed potential against a reference electrode, and the current flowing in the electron-transfer electrode is measured.

FIG.1

FIG.2

FIG.3.

FIG.4.

FIG.5.

FIG.6.

FIG.7.

FIG.8.

FIG.9.

FIG.10.

FIG.11.

(a)

(b)

$\mathrm{I}$ 1 $\mu$A

(c)

FIG.12.

0          +200          +400

E/mV  vs.SCE.

FIG.13a.

878

FIG.13b.

FIG.14.

FIG.15.

FIG . 16.

FIG.18.

FIG .17.

EP 0 351 892 A2

FIG.19.

EP 0 351 892 A2